(19) Europäisches Patentamt / European Patent Office / Office européen des brevets

(11) **EP 4 650 811 A1**

(12) **EUROPEAN PATENT APPLICATION**

(43) Date of publication:
**19.11.2025 Bulletin 2025/47**

(21) Application number: **24176188.1**

(22) Date of filing: **16.05.2024**

(51) International Patent Classification (IPC):
**G01R 33/563** (2006.01)    **G01R 33/567** (2006.01)
**G01R 33/50** (2006.01)    **G01R 33/56** (2006.01)
**G01R 33/561** (2006.01)

(52) Cooperative Patent Classification (CPC):
**G01R 33/5673; A61B 5/055; A61B 5/7285;**
**G01R 33/50; G01R 33/56325;** G01R 33/5602;
G01R 33/5617

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **SMINK, Jouke**
**Eindhoven (NL)**

(74) Representative: **Philips Intellectual Property &**
**Standards**
**High Tech Campus 52**
**5656 AG Eindhoven (NL)**

(54) **MODIFIED MRI PULSE SEQUENCE FOR CARDIAC T1-MAPPING**

(57) The invention provides for a medical system, a method of operating thereof and a computer program. The system comprises a magnetic resonance system (200) and a cardiac monitoring system (110) capturing cardiac phase data. A memory (320) holds machine executable instructions (330) and pulse sequence commands (340) causing magnetic resonance system (200) to acquire myocardial k-space data. The commands comprise at least one preparatory pulse portion (130) and multiple k-space acquisition portions (140). Executing instructions (330), computational system (302) processes cardiac phase data, identifies trigger signals (150) and causes data collection. A first preparatory pulse portion (131) follows a first k-space acquisition portion (141) after a first trigger signal (151) which occurs at a fixed acquisition delay (160) after the trigger. A second k-space acquisition portion (142) follows the pulse and a second trigger signal (152), the preparatory delay (170) between pulse and second acquisition exceeds the fixed acquisition delay.

Fig. 1

**Description**

TECHNICAL FIELD

The invention relates to magnetic resonance imaging, in particular to cardiac T1-mapping in scanners with a long latency cardiac synchronization technique.

BACKGROUND

In magnetic resonance imaging (MRI), cardiac T1 mapping is an essential technique for detecting tissue abnormalities that affect T1 relaxation times. The process involves the application of preparatory pulses to heart tissue typically followed by performing one or more k-space data acquisitions. A curve of a T1 relaxation model is fitted to the signal intensities reconstructed from the acquired k-space data corresponding to different delay times between the pulse application and the data acquisitions to estimate T1 values and generate a T1-map of the heart tissue. Expanding the range of delay times can improve the precision in calculating T1 values, allowing for a more accurate fit to the T1 relaxation model. Since time constraints due to cardiac motion complicate the T1 quantification, cardiac monitoring techniques are integrated into timing strategies to align k-space data acquisition with specific events in the cardiac cycle, typically the diastole phase. However, the range of available delay times is limited to the time window between the trigger signal and the event in the cardiac cycle associated with the trigger signal. This is particularly relevant for cardiac monitoring techniques such as photoplethysmography (PPG) due to the inherent latencies between the trigger signal and the event in the corresponding event in the cardiac cycle.

The journal article Daniel R. Messroghli, Radjenovic, S. Kozerke, D. M. Higgins, M. U. Sivananthan, and J. P. Ridgway (2004), Modified Look Locker Inversion Recovery (MOLLI) for High-Resolution T1 Mapping of the Heart, Magnetic Resonance in Medicine 52:141-146, doi: 10.1002/mrm.20110 discloses a pulse sequence scheme that measures myocardial T1 within a single breath-hold, involving selective data acquisition and merging data from multiple Look-Locker Inversion Recovery experiments into one dataset.

SUMMARY

The invention provides for a medical system, a method of operating a medical system, and a computer program in the independent claims. Embodiments are given in the dependent claims.

In one aspect a medical system is disclosed. The medical system comprises a magnetic resonance system, wherein the magnetic resonance system is configured for acquiring k-space data descriptive of a subject. The medical system further comprises a cardiac monitoring system. The cardiac monitoring system is configured for acquiring cardiac phase data descriptive of a cardiac phase of the subject. The medical system further comprises a memory storing machine-executable instructions and pulse sequence commands. The pulse sequence commands are configured to control the magnetic resonance system to acquire the k-space data according to a T1 mapping myocardial magnetic resonance protocol. The pulse sequence commands are comprising at least one preparatory pulse portion and multiple k-space acquisition portions. The medical system further comprises a computational system. Execution of the machine-executable instructions causes the computational system to continuously receive the cardiac phase data from the cardiac monitoring system and to continuously detect trigger signals in the received cardiac phase data correlated to a contraction of ventricles of the subject's heart. Execution of the machine-executable instructions further causes the computational system to perform acquisition of the k-space data using the pulse sequence commands. The pulse sequence commands are configured such that a first preparatory pulse portion of the at least one preparatory pulse portion occurs after a first k-space acquisition portion of the multiple k-space acquisition portions and at a pulse delay after a first trigger signal of the detected trigger signals, wherein the first k-space acquisition portion occurs at a fixed acquisition delay after the first trigger signal. The pulse sequence commands are further configured such that a second k-space acquisition portion of the multiple k-space acquisition portions immediately following the first k-space acquisition portion occurs after the first preparatory pulse portion and at the fixed acquisition delay after a second trigger signal of the detected trigger signals immediately following the first trigger signal, wherein a preparatory delay between the first preparatory pulse portion and the second k-space acquisition portion is longer than the fixed acquisition delay.

In another aspect a method of operating a medical system is disclosed, wherein the medical system comprises a magnetic resonance system configured for acquiring k-space data descriptive of a subject and a cardiac monitoring system configured for acquiring cardiac phase data descriptive of a cardiac phase of the subject. The method comprises continuously receiving cardiac phase data from a cardiac monitoring system and continuously detecting trigger signals in the received cardiac phase data correlated to a contraction of ventricles of the subject's heart. The method further comprises performing acquisition of k-space data using pulse sequence commands. The pulse sequence commands are configured such that a first preparatory pulse portion of at least one preparatory pulse portion occurs after a first k-space acquisition portion of the multiple k-space acquisition portions and at a pulse delay after a first trigger signal of the detected trigger signals, wherein the first k-space acquisition portion occurs at a fixed acquisition delay after the first trigger signal. The pulse sequence commands are further configured such that a second k-space acquisition portion of the multiple k-space acquisition portions immediately following the first k-space acquisition portion occurs after the first preparatory pulse portion and at the fixed acquisition delay after a second trigger signal of the detected trigger signals immediately following the first trigger signal, wherein a preparatory delay between the first preparatory pulse portion and the second k-space acquisition portion is longer than the fixed acquisition delay.

In another aspect a computer program is disclosed that comprises machine-executable instructions configured for causing a computational system to perform the aforementioned method of operating a medical system.

## BRIEF DESCRIPTION OF THE DRAWINGS

In the following, examples are described in greater detail making reference to the drawings in which:

> Fig. 1 illustrates an example of a medical system.
> Fig. 2 illustrates an exemplary pulse sequence according to a MOLLI protocol.
> Fig. 3 illustrates an exemplary diagram of an exponential T1 relaxation curve which has been fitted to data points derived using the pulse sequence of Fig. 2.
> Fig. 4 illustrates an exemplary pulse sequence according to a SASHA protocol.
> Fig. 5 shows a flow chart which illustrates a method of using the medical system of Fig. 1.

## DESCRIPTION OF EMBODIMENTS

Like numbered elements in these Figures are either equivalent elements or perform the same function. Elements which have been discussed previously will not necessarily be discussed in later Figures if the function is equivalent.

It is to be understood that numerals used to reference to or label individual elements, such as "first", "second", "third", e.g. in terms such as "first k-space acquisition portion" or "second trigger signal", do not imply a fixed chronological order, their only function is to reference to individual elements.

In an example a medical system comprises a magnetic resonance system, configured for acquiring k-space data descriptive of a subject. The k-space data may for example be descriptive of a part or all the subject's heart. The medical system further comprises a cardiac monitoring system configured for acquiring cardiac phase data descriptive of a cardiac phase of the subject. The cardiac monitoring system used herein comprises for example a detection unit, typically comprising a sensor and a processing unit, configured for detecting events in the subject's cardiac cycle. The detection unit may be able to detect the events in the cardiac cycle for example directly by measuring the heart's electrical activity, e.g., through electrocardiography (ECG) or vectorcardiography (VCG) techniques, which use electrodes placed on the subject's skin.

Another option for detecting the events in the cardiac cycle is for example by measuring the physiological changes caused by the events. For example, changes in blood volume and pressure may be measured via photoplethysmography (PPG), which uses a light source and a photosensor to detect color changes in the skin caused by blood volume changes in the microvascular bed of the skin tissue. The derived PPG waveform reflects the blood volume changes linked to the heart's pumping action. A photoplethysmogram may be realized for example by using a pulse pressure unit (PPU) which comprises a photosensor attached to the fingertip of the subject and a light source on the opposite side of the fingertip or by using a camera pointed for example at the subject's face. The data generated by the photosensor/camera is analyzed by a computational system for color changes, and the photoplethysmogram is created based on this analysis.

Other alternatives for detecting the events in the cardiac cycle include the use of an acoustic sensor for detecting the heartbeat or pilot tone detection for identifying changes in the magnetic field caused by the physical motion of the heart. An acoustic sensor may capture the sounds generated by the heart as it beats, such as the lub-dub sounds associated with the closing of the heart valves. Pilot tone detection, on the other hand, may use a radio frequency pulse generator to create a constant tone or signal within the magnetic resonance environment. As the heart moves and changes position within the magnetic field for example generated by the magnet of the magnetic resonance system, these movements modulate the pilot tone. These modulations may be detected for example by the coils of the magnetic resonance system.

The medical system further comprises a memory storing machine-executable instructions and pulse sequence commands. The pulse sequence commands are configured to control the magnetic resonance system to acquire the k-space data according to a T1 mapping myocardial magnetic resonance protocol. The pulse sequence commands comprise at least one preparatory pulse portion and multiple k-space acquisition portions to acquire the k-space data in synchronization with an event in the cardiac cycle, for example the diastole phase. This may optimize the timing of k-space data acquisition to coincide with periods of minimal heart movement. This synchronization may reduce motion artifacts and improve the accuracy and quality of the cardiac T1-maps. The at least one preparatory pulse portion of the pulse sequence commands is configured to cause the magnetic resonance system to apply a preparatory pulse to the subject's tissue. This preparatory pulse is designed to precondition the tissue for the acquisition of k-space data by adjusting its magnetization properties, typically the magnetic spins of protons due to the abundance of water and fat in the tissue. The multiple k-space acquisition portions of the pulse sequence commands are configured to cause the magnetic resonance system to perform k-space data acquisitions, for example by performing steady-state free precession (SSFP), black blood turbo spin echo (black blood TSE) read-outs or RF spoiled T1-weighted Turbo Field Echo (T1-TFE) read-outs.

For example, the preparatory pulse portions correspond to inversion pulses and may cause the magnetic resonance system to apply radiofrequency pulses that flip the magnetic spins in the tissue, usually the magnetic spins of the protons. Typically, these radiofrequency pulses are 180-degree inversion pulses. Such 180-degree inversion pulses are designed to invert the magnetization of spins in the tissue being imaged, effectively flipping the longitudinal magnetization from its equilibrium position into the opposite direction. Other flip angles are also possible. Following the inversion, the spins relax back towards their equilibrium state. This relaxation process is characterized by the T1 relaxation time, which is the time constant that describes how quickly the spins recover their equilibrium longitudinal magnetization. The T1 relaxation time may be defined as the time at which the magnetization has recovered to 63% of its value at equilibrium. Different tissues have different T1 relaxation times, allowing for the differentiation of tissue characteristics based on their relaxation properties.

Alternatively, or in addition, the preparatory pulse portions may correspond to saturation pulses and cause the magnetic resonance system to apply radiofrequency pulses designed to reduce the longitudinal magnetization towards zero, effectively saturating the spins in the tissue being imaged. These saturation pulses disrupt the equilibrium magnetization. Typically, 90-degree saturation pulses are utilized, but pulses with different flip angles may be used. After applying a saturation pulse, the spins return to their equilibrium state. This process is also characterized by the T1 relaxation time, thus allowing for the differentiation of tissue characteristics.

The medical system further comprises a computational system. Execution of the machine-executable instructions causes the computational system to continuously receive the cardiac phase data from the cardiac monitoring system and to continuously detect trigger signals in the received cardiac phase data correlated to a contraction of the ventricles of the subject's heart.

Execution of the machine-executable instructions further causes the computational system to perform acquisition of the k-space data using the pulse sequence commands. The pulse sequence commands in this example are configured such that a first preparatory pulse portion occurs after a first k-space acquisition portion and at a pulse delay after a first trigger signal of the detected trigger signals, wherein the first k-space acquisition portion occurs at a fixed acquisition delay after the first trigger signal. The fixed acquisition delay is the time interval between a trigger signal and a directly following k-space acquisition and may be specified as the time interval between the trigger signal and a specific point within the k-space acquisition, such as its mid-point, beginning, or end. Usually, the fixed acquisition delay is chosen based on the heart rate of the subject and may not be changed during a pulse sequence to ensure that all k-space acquisitions are performed during the same event in the cardiac cycle. Although the fixed acquisition delay generally remains constant throughout a pulse sequence to ensure consistency of the k-space acquisitions with respect to the cardiac cycle, it may be adjusted for example in response to significant heart rate variations during or between sequences, ensuring that all k-space acquisitions consistently align with the intended event in the cardiac cycle. The pulse sequence commands are further configured such that a second k-space acquisition portion of the multiple k-space acquisition portions immediately following the first k-space acquisition portion occurs after the first preparatory pulse portion at the fixed acquisition delay after a second trigger signal immediately following the first trigger signal. The preparatory delay between the first preparatory pulse portion and the second k-space acquisition portion is longer than the fixed acquisition delay.

This configuration of the pulse sequence commands may result in a first k-space acquisition being performed after a first trigger signal and a first preparatory pulse being applied after the first trigger signal but before a second trigger signal which is consecutive to the first trigger signal. Furthermore, after the second trigger signal a second k-space acquisition portion may be performed. In other words, between trigger signals one and two a k-space acquisition is performed before a preparatory pulse is applied, and between trigger signals two and three another k-space acquisition portion is performed. This second k-space acquisition is not restricted to being performed immediately after the trigger signal consecutive to the application of the preparatory pulse. It may also be performed at a later point in time. With this exemplary configuration the timing of the preparatory pulse may be independent from the second trigger signal and therefore decoupled from the time window between the second trigger signal and performing the second acquisition of k-space data.

The so-called preparatory delay between applying a preparatory pulse and acquiring k-space data is recorded and can also be referred to as T1 delay time, since it reflects the time interval allowing the spins in the tissue to relax towards equilibrium. To enable fitting of a curve of a relaxation model, at least k-space data acquired for two different T1 delay times is needed. Better mapping results are obtained when more than two k-space acquisitions with varying T1 delay times are performed. Typically, between 5 and 10 k-space acquisitions, each with different T1 delay times, are performed during a pulse sequence to ensure high accuracy of the fit. For example, multiple k-space acquisitions may be performed after one preparatory pulse has been applied. Typically, one k-space acquisition follows each subsequent trigger signal. Alternatively, for example multiple preparatory pulses may be applied, each followed by one or multiple k-space acquisitions with varying preparatory delays between each preparatory pulse and the following k-space acquisitions to acquire k-space data associated with a broad range of T1 delay times. The T1 delay times may be used to fit a curve of a theoretical T1 relaxation model to the signal intensities reconstructed from the acquired k-space data. This fitting process involves comparing the derived signal intensities at various T1 delay times with the theoretical signal intensity changes predicted by the recovery or decay associated with T1 relaxation. Typically, this recovery or decay is described with an exponential function. If the preparatory pulse and the following k-space acquisition are timed in a way that both occur between the same two consecutive trigger signals, the range of available T1 delay times is limited to the time interval between the trigger signal and the phase of the cardiac cycle during which the k-space data should be acquired. Therefore, this example may be beneficial because it may broaden the range of available T1 delay times, enabling T1 delay times not possible when both the preparatory pulse and the following k-space acquisition occur between the same two consecutive trigger signals. This broadened range of T1 delay times, in turn, may allow for a higher accuracy of the fit to the theoretical relaxation model. This may especially be beneficial for, but not limited to, medical systems with a cardiac monitoring system whose trigger signals have a significant latency for example more than 100 ms, to the detected event in the cardiac cycle. This latency reduces the available time between the trigger signal and the cardiac phase event, thereby limiting the T1 delay times achievable between a preparatory pulse and the subsequent k-space acquisition. For example, when comparing PPG to ECG/VCG directly, the PPG waveform's peak exhibits a latency of about 250 ms relative to the cardiogram's R wave. For an exemplary heart rate of 60 beats per minute, the diastole phase occurs around 650 ms after the cardiogram's R wave, wherein the duration of a complete cardiac cycle is around 1000 ms. Thus, in this exemplary scenario, the 250 ms latency of the PPG waveform's peak compared to the R wave effectively shortens the window for k-space acquisition from roughly 600 ms to 350 ms, significantly narrowing the range of available T1 delay times. By decoupling the timing of the preparatory pulse from this narrowed time window, e.g., T1 delay times between 50 ms and 600 ms, may be enabled. T1 delay times longer than 600 ms may be achieved by acquiring k-space data after a consecutive trigger signal.

In another example the T1 mapping myocardial magnetic resonance protocol is a modified look-locker inversion recovery (MOLLI) protocol. It is understood that every MOLLI protocol, in particular 5(3)3-MOLLI, 3(3)3(3)5-MOLLI, 4(1)3(1)2-MOLLI, 2(2)3(2)3-MOLLI is included. The MOLLI protocol may also be a Shortened MOLLI (ShMOLLI) protocol. This example may be beneficial because it may allow for a precise fitting of exponential curves to signal intensities reconstructed from the k-space data and may provide a higher flexibility in tailoring the process to the specific requirements of the subject due to the variable T1 delay times of the MOLLI protocol. Furthermore, the ability to adjust MOLLI sequences, including the flexibility to use shortened protocols like shMOLLI, may reduce scan times and may increase the robustness of the T1 mapping process against heart rate variability and other physiological fluctuations.

In another example the pulse sequence commands are divided into a first pulse sequence portion and a second pulse sequence portion, wherein the first pulse sequence portion comprises the first k-space acquisition portion, the first preparatory pulse portion, the second k-space acquisition portion and subsequent additional third k-space acquisition portions of the multiple k-space acquisition portions timed such that they occur at the fixed acquisition delay after respectively detected third trigger signals following the second trigger signals. Furthermore, the second pulse sequence portion comprises a second preparatory pulse portion of the preparatory pulse portions and a fourth k-space acquisition portion of the multiple k-space acquisition portions immediately following the second preparatory pulse portion. The pulse sequence commands are further configured such that the fourth k-space acquisition portion occurs at the fixed acquisition delay after a fourth trigger signal, and that the second preparatory pulse portion occurs after the fourth trigger signal and before the fourth k-space acquisition portion. Furthermore, the second pulse sequence portion further comprises fifth k-space acquisition portions timed such that they each occur at the fixed acquisition delay after respectively detected fifth trigger signals following the fourth trigger signal.

Execution of the pulse sequence commands of the first pulse sequence portion may result in the following chronological order of events at the beginning of the first pulse sequence portion: "first trigger signal - first k-space acquisition - preparatory pulse - second trigger signal - second k-space acquisition". This arrangement may enable short T1 delay times by decoupling the timing of the preparatory pulse from the second trigger signal which triggers the second k-space acquisition.

Execution of the pulse sequence commands may as well result in additional k-space acquisitions each being performed at the fixed acquisition delay after respectively detected trigger signals after the second k-space acquisition. This may result in the following chronological order of events: "first trigger signal - first k-space acquisition - first preparatory pulse - second trigger signal - second k-space acquisition - trigger signal - additional k-space acquisition - trigger signal - additional k-space acquisition - trigger signal - additional k-space acquisition". The additional k-space acquisitions after the second k-space acquisition may enable intermediate and long T1 delay times.

Execution of the pulse sequence commands of the second pulse sequence portion may result in the following chronological order of events: "trigger signal - second preparatory pulse - fourth k-space acquisition - trigger signal - k-space acquisition - trigger signal - k-space acquisition." Different to the first pulse sequence portion, execution of the pulse sequence commands of the second pulse sequence portion may result in a preparatory pulse directly followed by a k-space acquisition, wherein both occur between the same two consecutively detected trigger signals. This may enable very short T1 delay times. The k-space acquisitions following these two consecutively detected trigger signals may enable long T1 delay times.

The T1 delay times of the first pulse sequence portion may be interleaved with the T1 delay times of the second pulse sequence portion. This way k-space data may be acquired in a way that a broad range of different T1 delay times for fitting an exponential recovery curve is covered. Specifically, by interleaving the T1 delay times of the first and second pulse sequence portions, it may be possible to optimize the sampling of k-space data across the entire range of T1 delay times. For instance, the first pulse sequence portion may comprise five k-space acquisition portions in total and the second pulse sequence portion may comprise three k-space acquisition portions in total resulting in eight different T1 delay times. It is to be understood that the chronological order in which the two pulse sequence portions are arranged, as well as the number of k-space acquisition portions, are not restricted to the chronological order or numbers specified in this example. Also, between the two pulse sequence portions, there may be a pause between the pulse sequence portions to allow for physiological recovery of the subject's tissue before applying a preparatory pulse.

This example may be beneficial because it may improve the temporal resolution of the acquired k-space data by providing two distinct time windows to acquire k-space data following different T1 delay times for example within a single breath-hold of the subject, as compared to pulse sequence commands that consist of only one pulse sequence portion with one preparatory pulse portion.

In another example the pulse sequence commands are further configured such that the first k-space acquisition portion occurs before any other one of the k-space acquisition portions. This way the first k-space acquisition portion causes the magnetic system to acquire k-space data before any preparatory pulse is applied to the tissue. Consequently, the condition of the magnetic spins during this k-space acquisition is approximately equivalent to that of magnetic spins that have been relaxing for an infinite long period. This example may be beneficial since it may provide a data point corresponding to a very long T1 delay time with no extra time needed for the acquisition of this data point. This data point may be used for fitting an exponential relaxation curve to the signal intensity reconstructed from the k-space data to the point on the exponential relaxation curve corresponding to an infinite relaxation time and, therefore, improving the accuracy of the fit.

In another example the preparatory pulse portions are configured for causing the magnetic resonance imaging system to generate inversion pulses. Such inversion pulses are designed to invert the magnetization of spins in the tissue being imaged. This example may be beneficial because after the inversion, the spins begin to relax back towards their equilibrium state, which may enable the magnetic resonance system to track the relaxation process to determine T1 relaxation times precisely.

In another example the T1 mapping myocardial magnetic resonance protocol is a saturation-recovery single-shot acquisition (SASHA) protocol. This example may be beneficial because it may allow for absolute quantification of T1 values without the need for a heart-rate dependent correction, since the SASHA protocol has a reduced sensitivity to heart rate variability compared for example to the MOLLI protocol. This, in turn, may provide more accurate and consistent measurements across a wider range of heart rates.

In an example thereof the pulse sequence commands are comprising multiple preparatory pulse portions and the pulse sequence commands are further configured such that a third preparatory pulse portion of the multiple preparatory pulse portions occurs before the first k-space acquisition portion and every preparatory pulse portion is followed by a corresponding k-space acquisition portion to form a pair, wherein execution of the machine executable instructions causes the computational system to trigger the acquisition of the k-space data using the trigger signals such that the preparatory pulse portion of at least one pair is timed using the trigger signal preceding the trigger signal after which the respective corresponding k-space acquisition portion occurs.

By adding a third preparatory pulse portion that occurs before the first k-space acquisition portion, the first k-space acquisition portion can be paired with the third preparatory pulse portion. If the trigger signals are used for the acquisition of k-space data such that the preparatory pulse portion of at least one pair is timed using the trigger signal preceding the trigger signal after which the respective corresponding k-space acquisition portion occurs, the timing of the preparatory pulse is independent from the trigger signal timing the respective paired k-space acquisition. This may enable a broader range of T1 delay times, since the interval between the preparatory pulse and the k-space acquisition is not limited to the time window between the trigger signal timing the k-space acquisition and its subsequent k-space acquisition.

In an example thereof the pulse sequence commands are comprising at least three preparatory pulse portions and at least three k-space acquisition portions, wherein the pulse sequence commands are further configured such that the preparatory delay between the preparatory pulse portion and its corresponding k-space acquisition portion of at least one pair is shorter than the fixed acquisition delay. By comprising at least three preparatory pulse portions and at least three k-space acquisition portions at least three pairs of one preparatory pulse portion and one k-space acquisition portion can be formed. If the preparatory delay between the preparatory pulse portion and its corresponding k-space acquisition portion of at least one pair is shorter than the fixed acquisition delay, the preparatory pulse is applied during the time window between the trigger signal and its subsequent k-space data acquisition. This way shorter T1 delay times may be enabled resulting in an even broader range of T1 delay times.

In another example the pulse sequence commands are further configured such that every preparatory delay between any preparatory pulse portion and its corresponding k-space acquisition is unique. In other words, the T1 delay time of a pair of a preparatory pulse and its corresponding k-space acquisition is different to the T1 delay times of the other pairs. For instance, the T1 delay time of the first pair in the pulse sequence is the longest, and the T1 delay time progressively decreases with each subsequent pair. Different patterns of T1 delay times, e.g. starting with the shortest T1 delay time and progressively increasing the T1 delay time or interleaving T1 delay times are also possible.

In another example the k-space acquisition portions are configured to cause the magnetic resonance imaging system to perform steady-state free precession (SSFP) read-outs, including balanced steady-state free precession (b-SSFP) read-outs, black blood turbo spin echo (TSE) read-outs or RF spoiled T1-weighted Turbo Field Echo (T1-TFE) read-outs. This example may be beneficial because on one hand, SSFP read-outs may provide for high spatial resolution within a relatively short scan time. On the other hand, the black blood TSE read-outs may offer enhanced contrast between blood and surrounding tissues. Furthermore, T1-TFE read-outs may suppress background signals and enhance T1 contrast.

In another example the fixed acquisition delay is chosen such that the k-space acquisition portions occur during a diastole phase of the cardiac cycle, in particular a mid-diastole phase. This example may be beneficial because it may reduce motion artifacts in the k-space data due to the reduced physiological motion of the heart during diastole phase.

In another example execution of the machine executable instructions further causes the computational system to reconstruct a T1-weighted myocardial magnetic resonance image from the acquired k-space data according to the T1-weighted myocardial magnetic resonance protocol. This example may be beneficial because it may provide a T1-weighted myocardial magnetic resonance image with enhanced contrast and detail of the myocardial tissue, which may allow for improved differentiation of pathological changes from healthy myocardium.

In another example the cardiac monitoring system comprises at least one selected from the group comprising

- a photoplethysmography (PPG)-unit,
- a pulse pressure (PPU)-unit,
- an acoustic sensor, configured for detecting the subject's heartbeat, and
- a pilot tone-unit, configured for creating a pilot tone and analyzing modulations of the pilot tone.

This example may be beneficial because it may provide less invasive and simpler cardiac monitoring solutions, explicitly eliminating the requirement for electrode placement, unlike cardiac monitoring systems that rely on ECG/VCG.

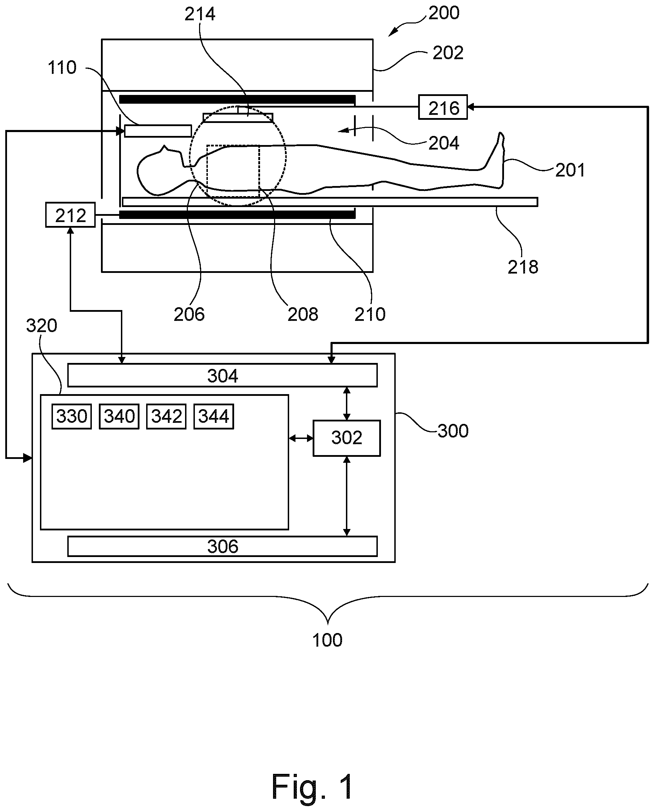

Fig. 1 illustrates an example of a medical system 100. The medical system 100 is shown as comprising a computer 300. The computer 300 may represent one or more computers located at one or more locations. The computer 300 comprises a computational system 302. The computational system 302 may represent one or more computational systems or computational cores that are located at one or more locations also. The computer 300 is further shown as containing an optional hardware interface 304 and an optional user interface 306 which are both in communication with the computational system 302. The hardware interface 304 may enable the computational system 302 to control other components of the medical system 100 if they are present. It may also enable the exchange of data with a network or other components of the medical system 100. The user interface 306 may enable an operator or user to control the operation and function of the medical system 100.

The medical system 100 is further shown as comprising a magnetic resonance system 200. The magnetic resonance imaging system comprises a magnet 202. The magnet 202 is a superconducting cylindrical type of magnet with a bore 204 through it. The use of different types of magnets is also possible; for instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject 201. The arrangement of the two sections area similar to that of a Helmholtz coil. Open magnets are popular because the subject 201 is less confined. Inside the cryostat of the cylindrical magnet there is a collection of superconducting coils.

Within the bore 204 of the cylindrical magnet 202 there is an imaging zone 206 where the magnetic field is strong and uniform enough to perform magnetic resonance imaging. A field of view 208 is shown within the imaging zone 206. The k-space data is typically acquired for the field of view 208. The region of interest could be identical with the field of view 208 or it could be a sub volume of the field of view 208. A subject 201 is shown as being supported by a subject support 218 such that at least a portion of the subject 201 is within the imaging zone 206 and the field of view 208.

Within the bore 204 of the magnet 202 there is also a set of magnetic field gradient coils 210 which are used for acquisition of preliminary k-space data to spatially encode magnetic spins within the imaging zone 206 of the magnet 202. The magnetic field gradient coils 210 are connected to a magnetic field gradient coil power supply 212. The magnetic field gradient coils 210 are intended to be representative. Typically, magnetic field gradient coils 210 contain three separate sets of coils for spatially encoding in three orthogonal spatial directions. A magnetic field gradient power supply supplies current to the magnetic field gradient coils 210. The current supplied to the magnetic field gradient coils 210 is controlled as a function of time and may be ramped or pulsed.

Adjacent to the imaging zone 206 is a radio frequency coil 214 for manipulating the orientations of magnetic spins within the imaging zone 206 and for receiving radio transmissions from spins also within the imaging zone 206. The radio frequency coil 214 may contain multiple coil elements. The radio frequency coil 214 may also be referred to as a channel or antenna. The radio-frequency coil 214 is connected to a radio frequency transceiver 216. The radio-frequency coil 214 and radio frequency transceiver 216 may be replaced by separate transmit and receive coils and a separate transmitter and receiver. It is understood that the radio-frequency coil 214 and the radio frequency transceiver 216 are representative. The radio-frequency coil 214 is intended to also represent a dedicated transmit antenna and a dedicated receive antenna. Likewise, the transceiver 216 may also represent a separate transmitter and receivers. The radio-frequency coil 214 may also have multiple receive/transmit elements and the radio frequency transceiver 216 may have multiple receive/transmit channels. The transceiver 216 and the gradient controller are shown as being connected to the hardware interface 304 of the computer 300.

The medical system 100 is further shown as comprising a cardiac monitoring system 110 which may comprise for example a light source and a camera suitable for PPG. The cardiac monitoring system 110 is also shown as being connected to the hardware interface 304 of the computer system. In this example the visual data captured by the camera is transmitted to the computer system where it is analyzed by the computational system 302 to extract cardiac phase data of the subject 201. Alternatively, the cardiac monitoring system 110 may also comprise a processing unit with a computational system 302 to analyze the visual data captured by the camera, extract the cardiac phase data from the visual data, and transmit the extracted cardiac phase data to the computer system of the medical device. For acquiring the cardiac phase data, instead of a camera and a light source pointed to the face of the subject 201, the cardiac monitoring system 110 may comprise a pulse pressure unit (PPU), for example realized by a pulse oximeter attached to the fingertip of the subject 201, utilizing a light source on one side of the finger and a photodetector positioned opposite the light source. The cardiac monitoring system 110 may also be realized by an acoustic sensor which is configured to capture the sounds generated by the heart. The cardiac monitoring system 110 may be realized by a radio frequency generator which sends a pilot tone through the tissue. In this case, modulations of the pilot tone that are caused by movements of the heart may be detected by the magnetic field gradient coils 210 or the radio frequency coil 214 of the magnetic resonance system 200.

The medical system 100 is further shown as comprising a memory 320. The memory 320 is intended to represent various types of memory which may be accessible to the computational system 302. In one example the memory 320 is a non-transitory storage medium. The memory 320 is shown as containing machine-executable instructions 330. The machine-executable instructions 330 may enable the computational system 302 to perform various computational and data manipulation and image processing tasks. The machine-executable instructions 330 may for example also be used or enable the computational system 302 to control other components of the medical system 100 via the optional hardware interface 304. Execution of the machine-executable instructions 330 by the computational system 302 may cause the computational system 302 to control the cardiac monitoring system 110 to continuously transmit cardiac phase data to the computational system 302. This cardiac phase data may be analyzed by the computational system 302 to continuously detect trigger signals. Determining the trigger signals generally depends on the cardiac monitoring technique. For example, when utilizing PPG, the trigger signal may be the peak of the PPG waveform. When utilizing ECG/VCG, the trigger signal may be the R wave of the cardiogram. When utilizing an acoustic sensor to detect the heartbeat, the trigger signal may be the S 1 (First Heart Sound) of the heart. When utilizing pilot tone, the trigger signal may be a specific pattern or change in the pilot tone modulation corresponding to significant heart movements.

The memory 320 is further shown as containing pulse sequence commands 340. The pulse sequence commands 340 may be configured to control the magnetic resonance system 200 to acquire the k-space data according to a T1 mapping myocardial magnetic resonance protocol. For this purpose, the pulse sequence commands 340 may comprise at least one preparatory pulse portion and multiple k-space acquisition portions (both not shown in Fig. 1), wherein the at least one preparatory pulse portion causes the magnetic resonance system 200 to apply a preparatory pulse the subject's tissue and the k-space acquisition portions cause the magnetic resonance system 200 to perform k-space acquisition portions. When the computational system 302 executes the machine-executable instructions 330 stored in the memory 320 the computational system 302 may control the magnetic resonance system 200 using the pulse sequence commands 340, specifically, e.g., to cause the radio-frequency coil 214 to apply a preparatory pulse to the subject's 201 tissue in the imaging zone 206 and to cause the magnetic field gradient coils 210 to perform k-space acquisitions. This may be realized by controlling the current supplied to the radio frequency coil 214 and the magnetic field gradient coils 210. The timing of the preparatory pulses and the k-space acquisitions may be based on the detection of trigger signals.

The k-space acquisition may be performed using steady-state free precession (SSFP) readouts through the computational system 302, including variations like a balanced Steady-State Free Precession (b-SSFP). Such SSFP readouts may be achieved by rapidly and repeatedly flipping the magnetic spins with a series of radio frequency pulses emitted by the radio frequency coil 214 at specific time intervals, creating a steady precession state. The precise timing and sequence of these radio frequency pulses, in conjunction with the manipulation of the gradient fields, may be carefully calibrated to maintain the spins in a steady-state equilibrium. This technique may result in high signal-to-noise ratio images with a high contrast. Furthermore, the computational system 302 may adjust the SSFP sequence parameters, such as flip angle and repetition time, to optimize image quality for the specific diagnostic requirements of the T1-weighted myocardial magnetic resonance imaging protocol. Alternatively, e.g., the k-space acquisition may be performed by using black blood Turbo Spin Echo (TSE) sequences, which can be utilized to suppress blood signal and enhance the visualization of myocardial tissue. Black blood TSE sequences employ a double inversion recovery prepulse that selectively nulls the signal from flowing blood, before acquiring the k-space data with a fast spin echo method. The computational system 302 may also modify parameters of the black blood TSE sequence, such as the inversion times and echo train length, to further enhance image quality and adapt to subject-specific conditions. Alternatively, e.g., the k-space acquisition may be performed using RF spoiled T1-weighted Turbo Field Echo (T1-TFE) read-outs, which may be particularly adept at enhancing myocardial tissue contrast by suppressing background signals. The computational system 302 may also modify parameters of the T1-TFE read-outs like may also modify parameters of the T1-TFE read-outs like echo time and repetition time to optimize contrast and spatial resolution.

Optionally, execution of the machine executable instructions may further cause the computational system 302 to reconstruct a T1-weighted myocardial magnetic resonance image from the acquired k-space data according to the T1-weighted myocardial magnetic resonance protocol. For example, suppose the T1-weighted myocardial magnetic resonance protocol is a MOLLI protocol. In that case, the T1-weighted myocardial magnetic resonance image may be reconstructed using different inversion relaxation delay times to fit a curve of an inversion relaxation models to the signal intensities reconstructed from the acquired k-space data. If for example the T1-weighted myocardial magnetic resonance protocol is a SASHA protocol, the T1-weighted myocardial magnetic resonance image may be reconstructed using different saturation relaxation delay times to fit curves of saturation relaxation models to the signal intensities reconstructed from the acquired k-space data to . Generally, the reconstruction process may involve iterative algorithms that adjust the parameters of a theoretical relaxation model to match the observed signal decay in the acquired k-space data. For this process for example optimization techniques like nonlinear least squares or machine learning approaches may be used. The process may iteratively refine the fit until the calculated T1 relaxation times for the myocardial tissue closely align with the signal intensities reconstructed from the k-space data. Thus, a detailed and clinically useful T1-weighted image may be produced.

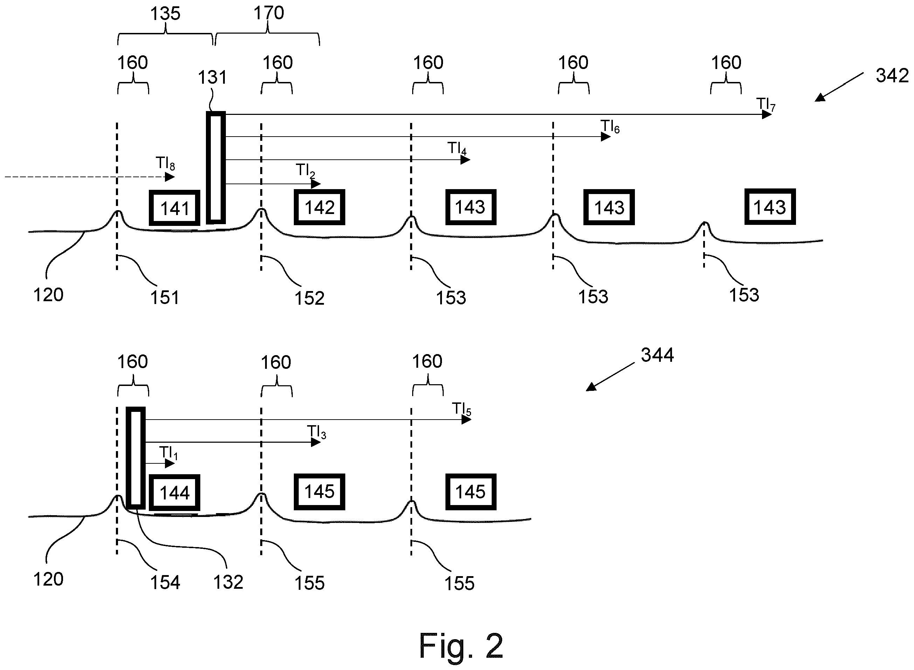

Fig. 2 illustrates an exemplary pulse sequence according to a MOLLI pulse sequence protocol. In this example, the pulse sequence is divided into a first pulse sequence portion 342 and a second pulse sequence portion 344. The first pulse sequence portion 342 has a length of five trigger signals, and the second pulse sequence portion 344 has a length of three trigger signals. There may also be additional trigger signals between the pulse sequence portions where no preparatory pulse is applied. The two pulse sequence portions may be executed in any chronological order. Dividing the pulse sequence into more than two portions or lengths of the two portions that differ from the shown example is also possible. In this example, for the sake of illustration but without limiting the scope, it is assumed that the preparatory pulse portion 131, 132 cause the magnetic resonance system 200 to perform inversion pulses, e.g. 180-degree inversion pulses.

Both pulse sequence portions are shown with an underlying PPG waveform 120. In each case, the PPG waveform 120 comprises peaks, wherein a trigger signal is associated with every peak and marked with a dashed vertical line. The first pulse sequence portion 342 may allow the computational system 302 to cause the magnetic resonance system 200 to perform a first k-space acquisition (corresponding to the first k-space acquisition portion 140) at the fixed acquisition delay 160 after the first trigger signal 151 has been detected. Furthermore, the first pulse sequence portion 342 may allow the computational system 302 to cause the magnetic resonance system 200 to apply a first preparatory pulse (corresponding to the first preparatory pulse portion 131) at a pulse delay 135 after the first trigger signal 151 and directly following the first k-space acquisition. After a second trigger signal 152 is detected after the first preparatory pulse, a second k-space acquisition portion (corresponding to the second k-space acquisition portion 142) may be performed. The T1 delay times in Fig. 1 are T1 inversion delay times, labelled $TI_1$ to $TI_8$, wherein a higher index number indicates a longer T1 inversion delay time. The T1 inversion delay time labelled $TI_2$ relates to the preparatory delay 170 between the first preparatory pulse portion 131 and the second k-space acquisition portion 142.

Every k-space acquisition portion occurs at the fixed acquisition delay 160 after a respective trigger signal. This way every k-space acquisition is performed during the same event in the cardiac cycle, in the case of Fig. 2 the diastole phase. This may ensure that the heart movement is minimal during a k-space acquisition is performed. In this example, the preparatory delay 170 is the delay between the end of the preparatory pulse portion and the mid-point of the k-space acquisition portion. Other form of delays like mid-point preparatory pulse portion to mid-point k-space acquisition portion or end of preparatory pulse portion to beginning of k-space acquisition are also possible.

After the second k-space acquisition portion 142 three third k-space acquisition portions 143 occur causing the magnetic resonance system 200 to perform three further k-space acquisitions each at the fixed acquisition delay 160 after a respective one of the third trigger signals 153. The T1 inversion delay times $TI_4$, $TI_6$ and $TI_7$ relate to the preparatory delay between the first preparatory pulse portion 131 and the respective one of these three third k-space acquisitions 143.

The T1 inversion delay times may be used for fitting a curve of an exponential inversion relaxation model to the signal intensities reconstructed from the k-space data to . The T1 delay time TIs, associated with the first k-space acquisition portion 141 of the first pulse sequence portion 342, may be seen as an almost infinite delay time since the k-space data is acquired without a prior preparatory pulse being applied to the tissue. For example, no inversion of the magnetic spins has been induced before this leading k-space acquisition. Consequently, the condition of the magnetic spins at this point is approximately equivalent to that of magnetic spins that have been relaxing for an indefinitely long period. As a result, the T1 delay time $TI_8$ may be used for fitting the exponential relaxation curve to the signal intensity reconstructed from the k-space data at a point in the curve corresponding to an infinite relaxation time.

Optionally, the fixed acquisition delay 160 may be chosen such that the k-space acquisition is performed during the diastole phase of the cardiac cycle for example mid- or late-diastole phase. For this, the computational system 302 may correlate patterns in the cardiac phase data, e.g., peaks in the PPG waveform 120, with appropriate timing for the k-space acquisition portions, ensuring that the acquisition coincides with for example mid-diastole phase. Additionally, algorithms, e.g., utilizing an autoregressive moving average (ARMA) model, may be employed to predict the timing of diastole phases by analyzing preceding cardiac cycles, potentially adjusting for heart rate variability to improve the precision of the acquisition timing.

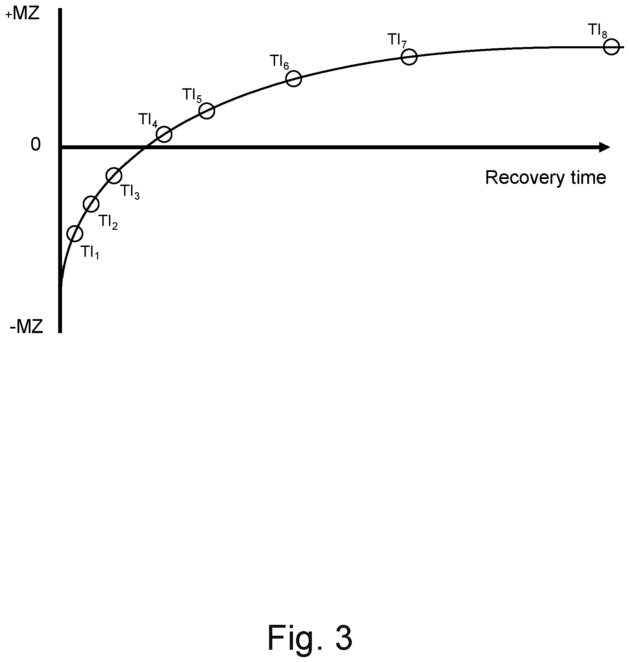

Fig. 3 illustrates an exemplary diagram with an exponential T1 relaxation curve which has been fitted to data points derived using the pulse sequence of Fig. 2 . The diagram demonstrates the relaxation of the longitudinal magnetization $M_z$ following a preparatory pulse characterized as an exponential relaxation process with a time constant T1. In this example, the data points may represent the longitudinal magnetization $M_z$ at various times of the relaxation process. The time constant is T1. In reference to Fig. 2, the longitudinal magnetization $M_z$ at a specific T1 delay time after the application of a preparatory pulse may be modeled by the formula:

$$M_z(TI) = M_0 \left( 1 - e^{-\frac{(TI)}{T_1}} \right)$$

Wherein $M_z$ is the longitudinal magnetization, $M_0$ is the longitudinal magnetization at its equilibrium state, TI is the T1 delay time, and $T_1$ is the time constant.

The longitudinal magnetization at the different T1 inversion delay times $TI_1$ to $TI_8$ may be reconstructed from the data of different k-space acquisitions. Ideally the data points are distributed along the entire exponential T1 relaxation curve, from its initial rapid relaxation phase to its eventual plateau, representing full relaxation. This distribution may allow for an accurate fit of the exponential relaxation model and, therefore, may allow for an accurate estimation of T1 relaxation times. The T1 inversion delay times $TI_1$ to $TI_7$ represent the intervals between applying a preparatory pulse and performing the subsequent k-space acquisition. The T1 inversion delay time $TI_8$ may serve as a reference point to fit the exponential relaxation curve to the longitudinal magnetization reconstructed from k-space data at a point in the plateau, where the magnetization is considered to have returned to its equilibrium state, effectively representing an infinite relaxation time.

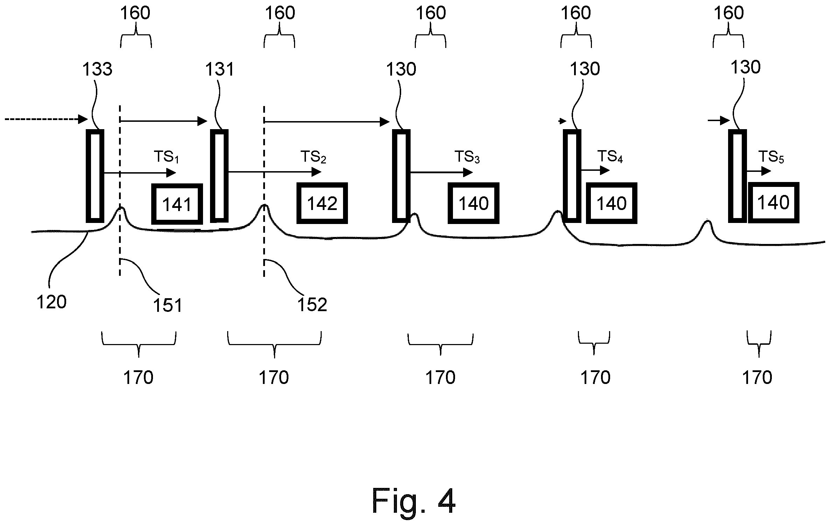

Fig. 4 illustrates an exemplary pulse sequence according to a SASHA pulse sequence protocol. The pulse sequence is shown with an underlying PPG waveform 120. The PPG waveform 120 comprises peaks, wherein a trigger signal is associated with every peak. The trigger signal is marked with a dashed vertical line for the two leftmost peaks. For clearness, only these lines are shown to indicate the trigger signal visually. It is to be understood that each of the other peaks also has a trigger signal associated with it. In this example, for the sake of illustration but without limiting the scope, it is assumed that the preparatory pulse portions 130, 131, 133 cause the magnetic resonance system 200 to apply saturation pulses. In this example, each preparatory pulse portion is paired with the following k-space acquisition portion. The third preparatory pulse portion 133 is paired with the first k-space acquisition portion 141 and the first preparatory pulse portion 131 is paired with the second k-space acquisition portion 142. The three preparatory pulse portions 130 are each paired with a respective one of the k-space acquisition portions 140. Every k-space acquisition portion occurs at the fixed acquisition delay 160 after a respective trigger signal. This way every k-space acquisition is performed during the same event in the cardiac cycle, in the case of Fig. 4 the diastole phase.

The preparatory delays 170 between a preparatory pulse portion and its corresponding k-space acquisition portion result in different T1 delay times, more specifically in different saturation delay times, labelled $TS_1$ to $TS_5$. The horizontal arrows positioned above the preparatory pulse portions indicate the pulse delay between the preparatory pulse portion and its preceding trigger signal. In the example of Fig. 4 the preparatory pulse corresponding to the third preparatory pulse portion 133 may be triggered by a trigger signal which is not shown in Fig. 4, which is indicated by the dashed line of the respective horizontal arrow. The preparatory pulse corresponding to the first preparatory pulse portion 131 is triggered by the first trigger signal 151 preceding the k-space acquisition corresponding to the first k-space acquisition portion 141. The preparatory pulse corresponding to the leftmost of the third preparatory pulse portions 130 may be triggered by the second trigger signal 152. This way the timing of the preparatory pulses corresponding to the first preparatory pulse portions 131, the third preparatory pulse portion 133 and the leftmost of the third preparatory pulse portions 130 is decoupled from the trigger signal that triggers the respective corresponding k-space acquisition. This may result in saturation delay times $TS_1$ to $TS_3$ that are longer than the fixed acquisition delay 160 that otherwise would be the upper limit for the length of the saturation delay time. Therefore, the range of available saturation delay times may be broadened.

In contrast, each of the preparatory pulses corresponding to the two rightmost preparatory pulse portions 130 and the respective paired k-space acquisition 140 may be triggered by the same respective trigger signal. This may result in saturation delay times $TS_4$ and $TS_5$ being shorter than the fixed acquisition delay 160. The combination of the short saturation delay times $TS_4$ and $TS_5$ and the longer saturation delay times $TS_1$ to $TS_3$ may enable a higher accuracy when fitting a curve of a T1 relaxation model to the data. This may especially be beneficial for, but not limited to, medical systems with a cardiac monitoring system whose trigger signals have a significant latency, e.g. a PPG system.

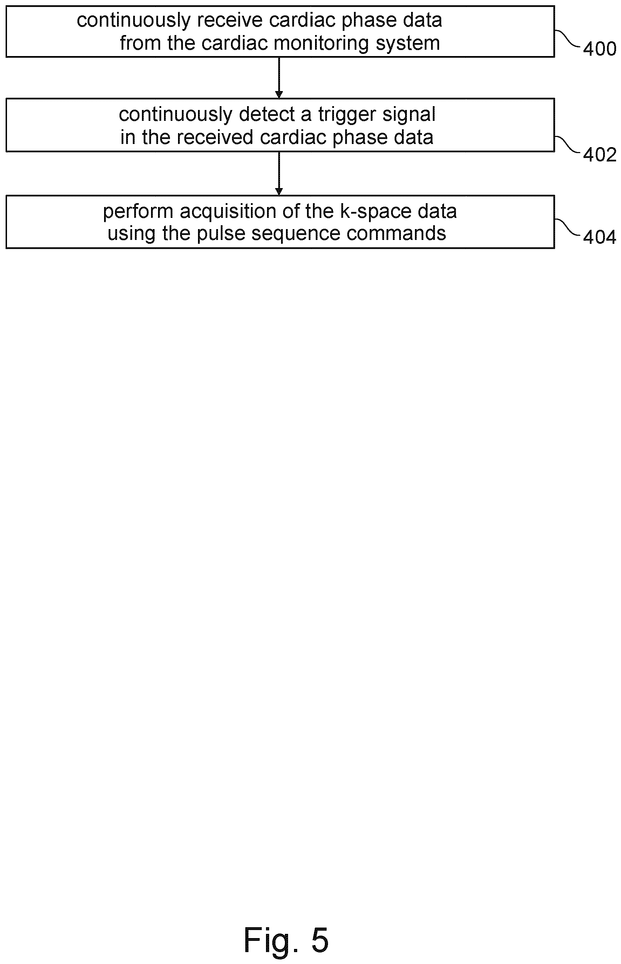

Fig. 5 shows a flow chart which illustrates a method of using the medical system 100 of Fig. 1. In step 400, the medical imaging system 100, or in this case the magnetic resonance system 200, is controlled by the computational system 302 to continuously receive the cardiac phase data from the cardiac monitoring system 110. In step 402, trigger signals 150 in the received cardiac phase data correlated to a contraction of ventricles of the subject's 201 heart are continuously detected. In step 404 acquisition of the k-space data using the pulse sequence commands 340 is performed, wherein in this case the pulse sequence commands 340 are configured such that a first preparatory pulse portion 131 occurs after a first k-space acquisition portion 141 and at a pulse delay 135 after a first trigger signal 151. Wherein the first k-space acquisition portion occurs at a fixed acquisition delay 160 after the first trigger signal 151. A second k-space acquisition portion 142 immediately following the first k-space acquisition portion 141 occurs after the first preparatory pulse portion 131 and at the fixed acquisition delay 160 after a second trigger signal 152 immediately following the first trigger signal 151, wherein a preparatory delay 170 between the first preparatory pulse portion 131 and the second k-space acquisition portion 142 is longer than the fixed acquisition delay 160.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein for example in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface 304 may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | medical system |
| 110 | cardiac monitoring system |
| 120 | PPG waveform |
| 130 | preparatory pulse portions |
| 131 | first preparatory pulse portion |
| 132 | second preparatory pulse portion |
| 133 | third preparatory pulse portion |
| 135 | pulse delay |
| 140 | k-space acquisition portions |
| 141 | first k-space acquisition portion |
| 142 | second k-space acquisition portion |
| 143 | third k-space acquisition portion |
| 144 | fourth k-space acquisition portion |
| 145 | fifth k-space acquisition portion |
| 150 | trigger signals |
| 151 | first trigger signal |
| 152 | second trigger signal |
| 153 | third trigger signal |
| 154 | fourth trigger signal |
| 155 | fifth trigger signal |
| 160 | fixed acquisition delay |
| 170 | preparatory delay |
| 200 | magnetic resonance system |
| 201 | subject |
| 202 | magnet |
| 204 | bore of magnet |
| 206 | imaging zone |
| 208 | field of view |
| 210 | magnetic field gradient coils |

212    magnetic field gradient coil power supply
214    radio-frequency coil
216    transceiver
218    subject support
300    computer
302    computational system
304    hardware interface
306    user interface
320    memory
330    machine-executable instructions
340    pulse sequence commands
342    first pulse sequence portion
344    second pulse sequence portion
400    continuously receive the cardiac phase data
402    continuously detect trigger signals
404    perform acquisition of the k-space data

**Claims**

1.  A medical system (100) comprising:

    - a magnetic resonance system (200), configured for acquiring k-space data descriptive of a subject (201);
    - a cardiac monitoring system (110), wherein the cardiac monitoring system is configured for acquiring cardiac phase data descriptive of a cardiac phase of the subject;
    - a memory (320) storing machine executable instructions (330) and pulse sequence commands (340), wherein the pulse sequence commands are configured to control the magnetic resonance system to acquire the k-space data according to a T1 mapping myocardial magnetic resonance protocol, wherein the pulse sequence commands are comprising at least one preparatory pulse portion (130) and multiple k-space acquisition portions (140);
    - a computational system (302), wherein execution of the machine executable instructions causes the computational system to:

        - continuously receive the cardiac phase data from the cardiac monitoring system;
        - continuously detect trigger signals (150) in the received cardiac phase data correlated to a contraction of ventricles of the subject's heart;
        - perform acquisition of the k-space data using the pulse sequence commands, wherein the pulse sequence commands are configured such that

            - a first preparatory pulse portion (131) of the at least one preparatory pulse portion occurs after a first k-space acquisition portion (141) of the multiple k-space acquisition portions and at a pulse delay after a first trigger signal (151) of the detected trigger signals,
            - the first k-space acquisition portion occurs at a fixed acquisition delay (160) after the first trigger signal,
            - a second k-space acquisition portion (142) of the multiple k-space acquisition portions immediately following the first k-space acquisition portion occurs after the first preparatory pulse portion and at the fixed acquisition delay after a second trigger signal (152) of the detected trigger signals immediately following the first trigger signal,

    wherein a preparatory delay (170) between the first preparatory pulse portion and the second k-space acquisition portion is longer than the fixed acquisition delay.

2.  The medical system of claim 1, wherein the T1 mapping myocardial magnetic resonance protocol is a modified look-locker inversion recovery (MOLLI) protocol.

3.  The medical system of claim 2, wherein the pulse sequence commands are divided into a first pulse sequence portion (342) and a second pulse sequence portion (344), wherein the first pulse sequence portion comprises the first k-space acquisition portion (141), the first preparatory pulse portion (131), the second k-space acquisition portion (142) and subsequent additional third k-space acquisition portions (143) of the multiple k-space acquisition portions timed such that they occur at the fixed acquisition delay (160) after respectively detected third trigger signals (153) following the second trigger signal (152), wherein the second pulse sequence portion comprises a second preparatory pulse portion (132) of the preparatory pulse portions and a fourth k-space acquisition portion (144) of the multiple k-space acquisition portions immediately following the second preparatory pulse portion, wherein the pulse sequence commands are further configured such that

        - the fourth k-space acquisition portion (144) occurs at the fixed acquisition delay after a fourth trigger signal (154)
        - the second preparatory pulse portion (132) occurs after the fourth trigger signal (154) and before the fourth k-space acquisition portion (144),

    wherein the second pulse sequence portion further comprises fifth k-space acquisition portions (145) timed such that they each occur at the fixed acquisition delay after respectively detected fifth trigger signals (155) following the fourth trigger signal.

4. The medical system of claim 3, wherein the pulse sequence commands are further configured such that the first k-space acquisition portion occurs before any other one of the k-space acquisition portions.

5. The medical system of any one of the claims 2-4, wherein the preparatory pulse portions correspond to inversion pulses.

6. The medical system of claim 1, wherein the T1 mapping myocardial magnetic resonance protocol is a saturation-recovery single-shot acquisition (SASHA) protocol.

7. The medical system of claim 6, wherein the pulse sequence commands are comprising multiple preparatory pulse portions and wherein the pulse sequence commands are further configured such that

- a third preparatory pulse portion (133) of the multiple preparatory pulse portions occurs before the first k-space acquisition portion (141),
- every preparatory pulse portion is followed by a corresponding k-space acquisition portion to form a pair,
wherein execution of the machine executable instructions causes the computational system to trigger the acquisition of the k-space data using the trigger signals such that the preparatory pulse portion of at least one pair is timed using the trigger signal preceding the trigger signal after which the respective corresponding k-space acquisition portion occurs.

8. The medical system of claim 7, wherein the pulse sequence commands are comprising at least three preparatory pulse portions (130, 131, 133) and at least three k-space acquisition portions (140, 141, 142), and wherein the pulse sequence commands are further configured such that the preparatory delay (170) between the preparatory pulse portion and its corresponding k-space acquisition portion of at least one pair is shorter than the fixed acquisition delay (160).

9. The medical system of any one of the claims 7 or 8, wherein the pulse sequence commands are further configured such that every preparatory delay between any preparatory pulse portion and its corresponding k-space acquisition is unique.

10. The medical system of any one of the preceding claims, wherein the k-space acquisition portions correspond to steady-state free precession (SSFP) read-outs, black blood turbo spin echo (black blood TSE) read-outs or RF spoiled T1-weighted Turbo Field Echo (T1-TFE) read-outs.

11. The medical system of any one of the preceding claims, wherein the fixed acquisition delay is chosen such that the k-space acquisition portions occur during a diastole phase of the cardiac cycle, in particular a mid-diastole phase.

12. The medical system of any one of the preceding claims, wherein execution of the machine executable instructions further causes the computational system to reconstruct a myocardial T1-map from the acquired k-space data according to the T1-weighted myocardial magnetic resonance protocol.

13. The medical system of any one of the preceding claims, wherein the cardiac monitoring system comprises at least one selected from the group comprising

- a photoplethysmography (PPG)-unit,
- a pulse pressure (PPU)-unit,
- an acoustic sensor, configured for detecting the subject's heartbeat, and
- a pilot tone-unit, configured for creating a pilot tone and analyzing modulations of the pilot tone.

14. A method of operating a medical system (100), wherein the medical system comprises a magnetic resonance system (200) configured for acquiring k-space data descriptive of a subject (201) and a cardiac monitoring system (110) configured for acquiring cardiac phase data descriptive of a cardiac phase of the subject, wherein the method comprises:

- continuously receiving (400) cardiac phase data from the cardiac monitoring system;
- continuously detecting (402) trigger signals in the received cardiac phase data correlated to a contraction of ventricles of the subject's heart;
- performing acquisition (404) of the k-space data using pulse sequence commands, wherein the pulse sequence commands are configured such that

- a first preparatory pulse portion of the at least one preparatory pulse portion occurs after a first k-space acquisition portion of the multiple k-space acquisition portions and at a pulse delay after a first trigger signal of the detected trigger signals,
- the first k-space acquisition portion occurs at a fixed acquisition delay after the first trigger signal,
- a second k-space acquisition portion of the multiple k-space acquisition portions immediately following the first k-space acquisition portion occurs after the first preparatory pulse portion and at the fixed acquisition delay after a second trigger signal of the detected trigger signals immediately following the first trigger signal, wherein a preparatory delay between the first preparatory pulse portion and the second k-space acquisition portion is longer than the fixed acquisition delay.

15. A computer program comprising machine executable instructions configured for causing a computational system to perform the method of claim 14.

Fig. 1

342

344

160 160 160 160 170 160 135 160

$TI_7$ $TI_6$ $TI_4$ $TI_2$ $TI_8$

143 143 143 142 131 141

153 153 153 152 151 120

160 160 160 160

$TI_5$ $TI_3$ $TI_1$

145 145 144

155 155 132 154 120

Fig. 2

Fig. 3

Fig. 4

continuously receive cardiac phase data
from the cardiac monitoring system ⌐400

continuously detect a trigger signal
in the received cardiac phase data ⌐402

perform acquisition of the k-space data
using the pulse sequence commands ⌐404

# Fig. 5

Europäisches
Patentamt

European
Patent Office

Office européen
des brevets

# EUROPEAN SEARCH REPORT

Application Number

EP 24 17 6188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DANIEL R. MESSROGHLI ET AL: "Modified Look-Locker inversion recovery (MOLLI) for high-resolution T1 mapping of the heart", MAGNETIC RESONANCE IN MEDICINE, vol. 52, no. 1, 28 June 2004 (2004-06-28), pages 141-146, XP055379973, US ISSN: 0740-3194, DOI: 10.1002/mrm.20110 * the whole document * | 1-15 | INV. G01R33/563 G01R33/567 G01R33/50 ADD. G01R33/56 G01R33/561 |
| A | PIECHNIK STEFAN K ET AL: "Shortened Modified Look-Locker Inversion recovery (ShMOLLI) for clinical myocardial T1-mapping at 1.5 and 3 T within a 9 heartbeat breathhold", JOURNAL OF CARDIOVASCULAR MAGNETIC RESONANCE, BIOMED CENTRAL LTD, LONDON UK, vol. 12, no. 1, 19 November 2010 (2010-11-19), page 69, XP021088932, ISSN: 1532-429X, DOI: 10.1186/1532-429X-12-69 * the whole document * | 1-15 | |
| A | CHOW KELVIN ET AL: "Saturation recovery single-shot acquisition (SASHA) for myocardial T1 mapping", MAGNETIC RESONANCE IN MEDICINE, vol. 71, no. 6, 23 July 2013 (2013-07-23), pages 2082-2095, XP093205460, US ISSN: 0740-3194, DOI: 10.1002/mrm.24878 * the whole document * | 1-15 | |

TECHNICAL FIELDS
SEARCHED       (IPC)

G01R

-/--

The present search report has been drawn up for all claims

| Place of search | Date of completion of the search | Examiner |
|---|---|---|
| Munich | 24 October 2024 | Raguin, Guy |

**EP 4 650 811 A1**

# EUROPEAN SEARCH REPORT

Application Number

EP 24 17 6188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TONET ELISABETTA ET AL: "Current evidence on the diagnostic and prognostic role of native T1 mapping in heart diseases", TRENDS IN CARDIOVASCULAR MEDICINE, ELSEVIER SCIENCE, NEW YORK, NY, US, vol. 31, no. 7, 7 August 2020 (2020-08-07), pages 448-454, XP086791874, ISSN: 1050-1738, DOI: 10.1016/J.TCM.2020.08.001 [retrieved on 2020-08-07] * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 24 October 2024 | Raguin, Guy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DANIEL R. MESSROGHLI ; RADJENOVIC, S. KOZERKE ; D. M. HIGGINS ; M. U. SIVANANTHAN ; J. P. RIDGWAY**. Modified Look Locker Inversion Recovery (MOLLI) for High-Resolution T1 Mapping of the Heart,. *Magnetic Resonance in Medicine*, 2004, vol. 52, 141-146